## Europäisches Patentamt
## European Patent Office
## Office européen des brevets

(19)

(11) Veröffentlichungsnummer: **0 263 789 B1**

(12)
# EUROPÄISCHE PATENTSCHRIFT

(45) Veröffentlichungstag der Patentschrift: **12.06.91**

(51) Int. Cl.⁵: **A61M 5/14, F16L 37/28**

(21) Anmeldenummer: **87810555.0**

(22) Anmeldetag: **25.09.87**

(54) **Zweiteilige Kupplungseinrichtung zum Austausch von flüssigen und gasförmigen Medien.**

(30) Priorität: **29.09.86 CH 3890/86**

(43) Veröffentlichungstag der Anmeldung:
**13.04.88 Patentblatt 88/15**

(45) Bekanntmachung des Hinweises auf die
Patenterteilung:
**12.06.91 Patentblatt 91/24**

(84) Benannte Vertragsstaaten:
**BE DE ES FR GB IT NL SE**

(56) Entgegenhaltungen:
**FR-A- 2 523 448**
**US-A- 4 334 551**

(73) Patentinhaber: **Contempo Products, P. Herrli**
**Alpenstrasse 15a**
**CH-2502 Biel(CH)**

(72) Erfinder: **Herrli, Peter**
**Alpenstrasse 15a**
**CH-2502 Biel(CH)**

(74) Vertreter: **Schweizer, Hans et al**
**Bovard AG Patentanwälte VSP Optingen-**
**strasse 16**
**CH-3000 Bern 25(CH)**

EP 0 263 789 B1

Rank Xerox (UK) Business Services

## Beschreibung

Die Erfindug betrifft eine zweiteilige Kupplungseinrichtung gemäss dem Oberbegriff des Patentanspruches 1. Eine deratige Kupplungseinrichtung geht aus FR-A-2 523 448 hervor.

Eine solche zweiteilige Kupplungseinrichtung wird insbesondere in einem System zum Ausführen eines ambulanten dialytischen Entzuges von durch geschädigte Nieren eines Patienten ausgeschiedenen Stoffwechselprodukten eingesetzt. In einem solchen Fall wird aus der Bauchhöhle des Patienten verbrauchtes Dialysat abgeführt und nachher in dieselbe frisches Dialysat eingeführt.

Eine ambulante Peritonealdialyse kann von einem nierengeschädigten Patienten selber ohne Unterbrechung während 24 Stunden pro Tag und 7 Tage in der Woche ausgeführt werden, z.B. bei der CAPD mindestens 4 mal pro Tag. Das in die Bauchhöhle des Patienten aus einem Beutel einzuführende frische Dialysat hat ein Volumen von meistens zwei Litern. Das verbrauchte Dialysat wird in einen leeren Beutel abgeführt. Durch die Peritonealdialyse werden von den geschädigten Nieren des Patienten ausgeschiedene Stoffwechselsubstanzen aus dem Körper des Patienten entfernt, wobei er sich während den Wechselzyklen seiner üblichen täglichen Tätigkeit widmen kann.

In dem System zum Ausführen einer Peritonealdialyse transportieren der Katheter (üblicherweise Tenckhoff-Katheter) und der angeschlossene Anschlussschlauch das verbrauchte Dialysat in einer Richtung und das frische Dialysat in der Gegenrichtung. Die Kupplungseinrichtung ist in diesem System zwischengeschaltet. Der Funktionsablauf ist immer so, dass beim Dialysatwechsel zuerst das verbrauchte Dialysat aus der Bauchhöhle des Patienten abfliesst und nachher über das gleiche Katheter-und Schlauchsystem frisches Dialysat in umgekehrter Richtung einfliesst.

Es ist a priori anzunehmen, dass die Luft ausserhalb der Kupplungseinrichtung kontaminiert ist, während die im Katheter und in der Kupplungseinrichtung befindlichen Flüssigkeiten als steril angenommen werden.

Um das Problem der Verbreitung einer Kontamination durch Bakterienkeime in der bei der Peritonealdialyse verwendeten Vorrichtung zu verhindern, hat die Firma Fresenius AG, Oberurstel, BRD, ein CAPD-Safe-Lock 5B entwickelt. Im ersten Kupplungsteil dieser zweiteiligen Kupplungseinrichtung, in welcher der Katheter bzw. sein Verlängerungsstück endet, ist ein Durchflusskanal gebildet, in welchem eine federbelastete Klappe untergebracht ist. Der zweite Kupplungsteil, in welchem sowohl der Schlauch zum Zuführen des frischen Dialysats als auch der Schlauch zum Abführen des verbrauchten Dialysats untergebracht sind, ist auf den ersten Kupplungsteil aufschraubbar und mittels eines Bajonettsystems arretierbar.

In der ersten Bajonettstellung werden die Anschlussteile durch ein Volumen des frischen Dialysats umspült, welches dann in den Schlauch für das verbrauchte Dialysat abgeführt wird. In der zweiten Bajonettstellung wird durch den vorstehenden Endteil des Schlauches zum Zuführen des frischen Dialysats die federbelastete Klappe von ihrer Sitzstelle abgedrückt, so dass das verbrauchte Dialysat aus der Bauchhöhle des Patienten in den zuständigen Schlauch abgeführt werden kann; dabei bleibt die Oeffnung des Endteiles des Schlauches zum Zuführen des frischen Dialysats durch die Klappe abgeschlossen. In der dritten Bajonettstellung wird die Klappe von der Mündung des Endteiles des Schlauches zum Zuführen des frischen Dialysats abgeklappt und das frische Dialysat kann in den Katheter fliessen.

Es besteht weiter die Gefahr, dass die in der Kupplungseinrichtung beim Ankoppeln eingeschlossene Luft zusammen mit dem frischen Dialysat in die Bauchhöhle des Patienten gelangt. Des weiteren wird, wenn eine turbulente Strömung auftritt, Fibrin ausgeschieden, das mit dem verbrauchten Dialysat abzuführen ist. Dieses Fibrin kann sich auf der Schraubenfeder und der Klappe absetzen und den Durchflusskanal verstopfen. Dadurch werden die Feder und die Klappe in ihrer Funktion behindert, was dazu führen kann, dass die Klappe den Ausgang aus dem Endteil des Schlauches zum Führen des frischen Dialysats nicht dicht abschliesst.

In der FR-A-2,523,448 wird eine Kupplungseinrichtung beschrieben, bei welcher durch Zusammenstecken zweier Kupplungshälften ein Strömungsweg zum Durchleiten der Flüssigkeit freigegeben wird. Das Verschliessen des Strömungsweges im getrennten Zustand der Kupplung erfolgt mittels zwei hinterinanderliegender Dichtscheiben. Diese Dichtscheiben bestehen aus einem elastischen Material und sind mit sternförmig angeordneten Schlitzen versehen. Beim Zusammenfügen der Kupplungshälften werden diese Dichtscheiben von einem eindringenden Zentralrohr geöffnet.

Der sich damit ergebende Durchflusskanal ist auch hier nicht frei von hervorstehenden Kanten und gewährleistet somit keine turbulenzfreie Strömung. Neben der dadurch verursachten Gefahr einer Fibrinausscheidung gibt auch diese Einrichtung keine Sicherheit, dass mit dem frischen Dialysat keine Luft in die Bauchhöhle des Patienten gelangt.

Der im Patentanspruch 1 angegebenen Erfindung liegt die Aufgabe zugrunde, eine zweiteilige Kupplungseinrichtung zu schaffen, bei welcher die Folgen einer eventuellen Kontamination der sie durchlaufenden Flüssigkeiten ausgeschlossen werden. Jegliche Aussenluft, die beim Kupplungsvorgang in die Kupplungseinrichtung gelangt, darf nicht weiter in den Katheter oder in die Bauchhöhle des Patienten gelangen und muss mit dem Auslaufen des verbrauchten Dialysats aus der Kupplungseinrichtung vollständig evakuiert werden. Alle festen Teile, die gegebenenfalls vom Patienten ausgeschieden und mit dem verbrauchten Dialysat transportiert werden, sollen mit Sicherheit die Kupplungseinrichtung passieren. Die Kupplungseinrichtung soll einfach zu bedienen sein, damit Fehlmanipulationen wirksam verhindert werden.

Die Aufgabe wird erfindungsgemäss durch die Merkmale des kennzeichnenden Teiles des Patentanspruches 1 gelöst. Die zweiteilige Kupplungseinrichtung wird erfindungsgemäss in einem System zum Ausführen einer Peritonealdialyse bei Patienten mit geschädigten Nieren verwendet.

Die Erfindung wird nachstehend anhand der Zeichnung beispielsweise näher erläutert. Es zeigen

Fig. 1 einen Längsschnitt durch die beiden Kupplungshälften eines Ausführungsbeispieles der erfindungsgemässen Kupplungseinrichtung, wobei in der oberen Hälfte der Figur nur der eine Kupplungsteil und in der unteen Hälfte der Figur die beiden zusammengesetzten Kupplungsteile dargestellt sind,

Fig. 2 eine perspektivische auseinandergezogene Darstellung des ersten Kupplungsteiles und des zweiten Kupplungsteiles,

Fig. 3 einen Schnitt entlang der Linie III-III der Fig. 1,

Fig. 4 einen Schnitt entlang der Linie IV-IV der Fig. 1, wobei die beiden Kupplungsteile zusammengesetzt sind,

Fig. 5 einen Schnitt entlang der gleichen Linie IV-IV der Fig. 1, wobei der zweite Kupplungsteil entfernt ist und

Fig. 6 eine schematische Ansicht eines Systems zum Ausführen der ambulanten Peritonealdialyse.

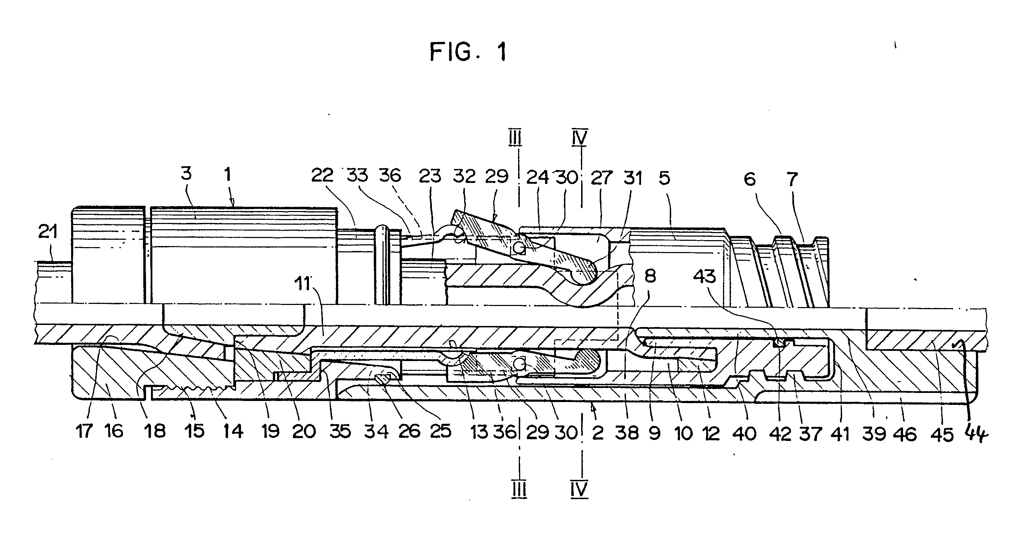

Das in den Figuren dargestellte Ausführungsbeispiel der erfindungsgemässen Kupplungseinrichtung umfasst einen ersten Kupplungsteil 1 und einen zweiten Kupplungsteil 2 und ist in der Fig. 1 teilweise im Schnitt dargestellt. Die obere Hälfte der Fig. 1 zeigt nur den ersten Kupplungsteil 1 und die untere Hälfte der Fig. 1 zeigt den auf den ersten Kupplungsteil aufgesetzten zweiten Kupplungsteil 2. Der erste Kupplungsteil umfasst eine Muffe 3, einen daran anschliessenden mehrfach abgesetzten Ansatz 4 und eine den Ansatz 4 mehrheitlich umgebende Hülse 5. Das dem Ansatz 4 abgewandte Ende der Hülse 5 weist eine mit einem Aussengewinde 6 versehene Verlängerung 7 auf. Anschliessend an die Innenseite der Verlängerung 7 ist eine konzentrisch zur Aussenwand 8 der Hülse 5 angeordnete Innenwand 9 vorhanden, welche Wände einen ringförmigen Raum 10 begrenzen. In den Raum 10 ragt das eine Ende eines elastischen Hohlkörpers, beispielsweise ein Schlauchstück 11 hinein, welches Ende mit einem Pressring 12 in dem ringförmigen Raum 10 befestigt ist. Das Schlauchstück 11 erstreckt sich durch einen zentralen Längsdurchlass 13 des Ansatzes 4 und der Muffe 3 und endet etwa in der Mitte der Muffe 3.

Das dem Ansatz 4 abgewandte Ende der Muffe 3 ist ausgedreht und weist ein Innengewinde 14 auf, in welches das Aussengewinde 15 eines Abschlussgliedes 16 des ersten Kupplungsteiles 1 eingeschraubt ist. Das Abschlussglied 16 weist eine Zentralbohrung 17 auf, deren Durchmesser von aussen nach innen zunimmt. Innerhalb der Muffe 3 ist ein Stützring 18, welcher teilweise in den Längsdurchlass 13 und in die Zentralbohrung 17 hineinragt, konzentrisch angeordnet. Die innere Hälfte des Stützringes 18 weist eine gleichmässige Dicke auf und der Aussendurchmesser der äusseren Hälfte des Stützringes 18 nimmt von aussen nach innen zu und bildet etwa in der Mitte eine Auflageschulter 19, an welcher das in die Muffe 3 ragende Ende des Schlauchstückes 11 anliegt.

Der in die Muffe 3 ragende Endbereich des Schlauchstückes 11 ist von einem Klemmring 20 mit keilförmigem Querschnitt umgeben. Der Klemmring 20 wird durch das Abschlussglied 16, bezogen auf die Fig. 1, nach rechts gepresst, wobei der zwischen dem Klemmring 20 und der inneren Hälfte des Stützringes 18 mit der gleichmässigen Dicke befindliche Teil des Schlauchstückes 11 innerhalb der Muffe 3 durch Klemmwirkung festgehalten wird.

Das distale Ende eines Katheters 21 (z.B. Tenckhoff-Katheter) erstreckt sich durch die Zentralbohrung 17 des Abschlussgliedes 16 und umgibt jene Hälfte des Stützringes 18 dessen Aussendurchmesser von aussen nach innen zunimmt. Dadurch wird das distale Ende des Katheters 21 ebenfalls durch Klemmwirkung innerhalb der Muffe 3 festgehalten.

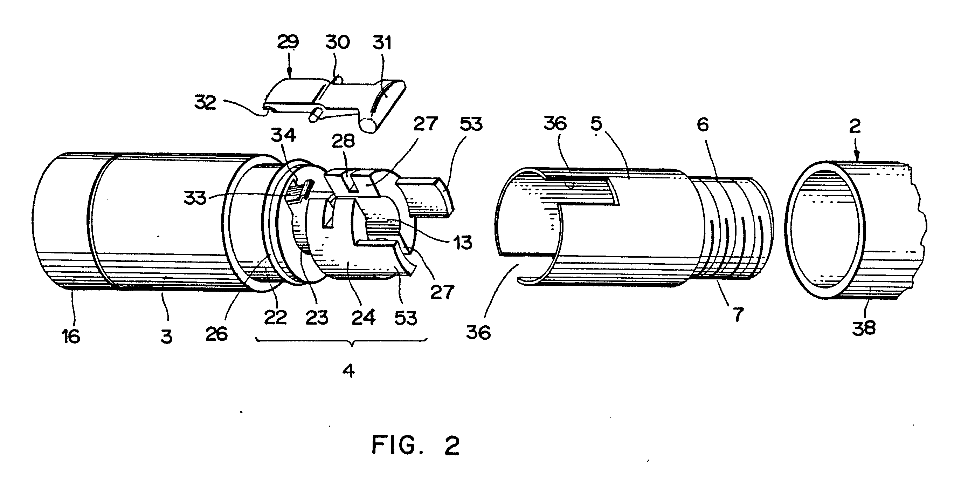

Der Ansatz 4 weist drei Abschnitte 22, 23 und 24 mit unterschiedlichen Aussendurchmessern auf, siehe auch Fig. 2. Der Aussendurchmesser des ersten Abschnittes 22 ist kleiner als jener der Muffe 3, jedoch grösser als der Aussendurchmesser des dritten Abschnittes 24, der über den zweiten Abschnitt 23 mit dem ersten Abschnitt 22 verbunden ist. Der Aussendurchmesser des zweiten Abschnittes 23 ist kleiner als jener des dritten Abschnittes 24.

Im ersten Abschnitt 22 ist eine Umfangsnut 25 eingelassen, in welcher sich ein Dichtungsring 26 befindet. An zwei einander gegenüberliegenden Stellen des dritten Abschnittes 24 sind sowohl radial als auch achsial verlaufende Ausnehmungen 27 vorhanden. Einschnitte 28 erstrecken sich quer zu den Ausnehmungen 27. In jeder der Ausnehmungen 27 ist je ein um eine quer zur Längsachse der Kupplungseinrichtung verlaufenden Achse schwenkbare Wippe 29 angeordnet. Jede Wippe 29 weist zwei seitlich vorstehende Lagerzapfe 30 auf, die in den Einschnitten 28 lagern (Fig. 2).

Das über den dritten Abschnitt 24 vorstehende Ende der Wippe 29 ist als Klemmbacke 31 ausgebildet, die zum Einwirken auf das Schlauchstück 11 bestimmt ist. Das andere Ende der Wippe 29 weist einen bogenförmigen Ausschnitt 32 zum Zusammenwirken mit dem freien Ende eines Federelementes 33 auf. Das Federelement 33 erstreckt sich im wesentlichen parallel zur Längsachse des ersten Kupplungsteiles 1 durch eine in der genannten Längsrichtung verlaufende Führungsnut 34 innerhalb des ersten Abschnittes 22 des Ansatzes 4 und der Muffe 3. Der dem freien Ende des Federelementes 33 gegenüberliegende Endbereich desselben ist zweimal abgebogen und ist zwischen einer Auflageschulter 35 innerhalb der Muffe 3 und der kleinen Stirnseite des Klemmringes 20 festgeklemmt. Das Federelement 33 ist so vorgespannt, dass das freie Ende desselben das mit dem bogenförmigen Ausschnitt 32 versehene Ende der Wippe 29 radial nach aussen drückt.

Die Hülse 5 besitzt zwei einander gegenüberliegende Ausschnitte 36, durch welche hindurch je ein Teil der zugehörigen Wippe ragt, wenn der zweite Kupplungsteil 2 vom ersten Kupplungsteil 1 abgenommen ist. Der Aussendurchmesser des dritten Abschnittes 24 des Ansatzes 4 ist angenähert gleich gross wie der Innendurchmesser der Hülse 5, so dass diese mit Presssitz über den dritten Abschnitt 24 aufgeschoben und mit diesem starr verbunden ist.

Der zweite Kupplungsteil 2 besitzt ein Innengewinde 37 und einen daran anschliessenden hohlen Vorsprung 38. Beim Zusammensetzen der beiden Kupplungsteile 1 und 2 wird das Innengewinde 37 des Kupplungsteiles 2 auf das Aussengewinde 6 auf der Verlängerung 7 der Hülse 5 aufgeschraubt. Der Innendurchmesser des hohlen Vorsprungs 38 ist etwas grösser als der Aussendurchmesser des Abschnittes 22 des Ansatzes 4 und der Aussendurchmesser der Hülse 5. Die Länge des Vorsprunges 38 ist so gewählt, dass bei vollständig auf das Aussengewinde 6 aufgeschraubten zweiten Kupplungsteil 2 das Ende des Vorsprunges 38 fast bis zur Muffe 3 reicht. Dadurch ist gewährleistet, dass der Endbereich des Vorsprunges 38 mit dem Dichtungsring 26 in der Nut 35 des Abschnittes 22 zusammenwirkt. Der dem Vorsprung 38 abgewandte Endbereich des zweiten Kupplungsteiles 2 besitzt anschliessend an das Innengewinde 37 eine radial nach innen vorstehende Rippe 39, die eine zum Vorsprung 38 konzentrisch angeordnete zylindrische Wand 40 trägt. Der Innendurchmesser der zylindrischen Wand 40 ist gleich dem Innendurchmesser des Schlauchstückes 11. Die zylindrische Wand 40, das Innengewinde 37 und ein Teil des Vorsprunges 38 begrenzen eine ringförmige Kammer 41, in welche ein Teil der Hülse 5 samt dem Aussengewinde 6 auf der Verlängerung 7 hineinragt.

Auf der Innenseite der Verlängerung 7 der Hülse 5 ist eine Umfangsnut 42 eingelassen in die ein Dichtungsring 43 eingelegt ist. Der Dichtungsring 26 beim Abschnitt 22 und der Dichtungsring 43 bei der Verlängerung 7 bilden eine wirksame Bakteriensperre, d.h. es wird verhindert, das Bakterien von aussen in den Durchflusskanal gelangen. Die Bakteriensperre ist besonders dann voll wirksam, wenn die Distanz in achsialer Richtung zwischen dem Dichtungsring 26 und der Schwenkachse der Wippe 29 kleiner ist als die Distanz zwischen dem freien Ende der zylindrischen Wand 40 und dem Dichtungsring 43.

Die Bohrung 44 im äusseren Endbereich des zweiten Kupplungsteiles 2, d.h. zwischen der Rippe 39 und dem äusseren Ende des zweiten Kupplungsteiles 2, weist einen grösseren Innendurchmesser auf als jener der zylindrischen Wand 40. In diese Bohrung 44 ist ein Schlauch 45 mittels Klebstoff befestigt. Zum Verbessern der Griffigkeit sind auf der Aussenseite des genannten Endbereiches des zweiten Kupplungsteiles in der Längsrichtung verlaufende und über den Umfang gleichmässig verteilte Rippen 46 vorhanden. Vorzugsweise können ähnliche Rippen auch auf der Aussenseite der Muffe 3 vorhanden sein.

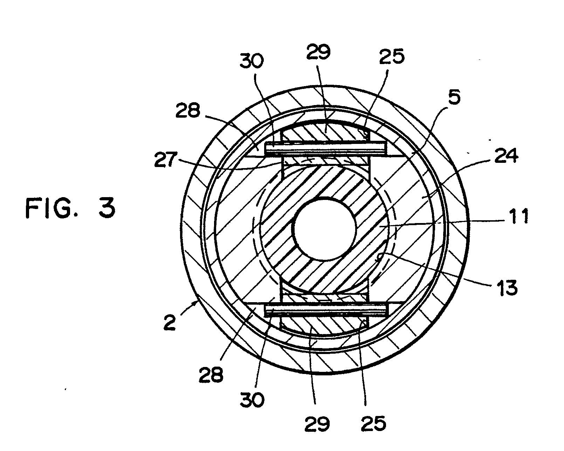

Die Fig. 3 zeigt einen Schnitt entlang der Linie III-III der Fig. 1 durch die vollständige Kupplungseinrichtung, wobei die beiden Teile 1 und 2 zusammengesetzt sind. In diesem Zustand ist das Schlauchstück 11 nicht zusammengepresst und der Durchlasskanal der Kupplungseinrichtung weist durchgehend den gleichen runden Querschnitt auf, weil die Innendurchmesser des Katheters 21, des Stützringes 18, des Schlauchstückes 11, der zylindrischen Wand 40 und des Schlauches 45 alle gleich gross sind. Deshalb ist die Strömung der Flüssigkeit durch die Kupplungseinrichtung laminar und es kann sich kein Fibrin bilden und ausserdem besteht keine Gefahr, dass allfällige feste Teile, welche mit dem Dialysat durch den Katheter 21 transportiert werden, die Kupplungseinrichtung verstopfen.

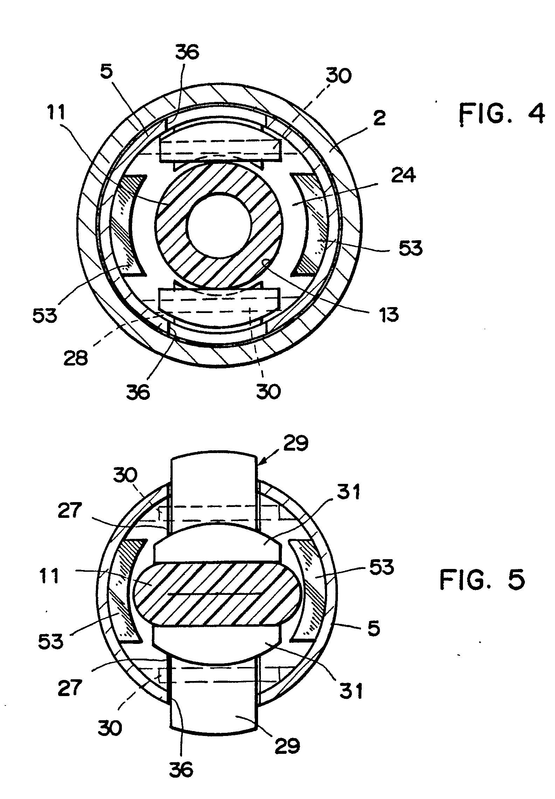

Die Fig. 4 zeigt einen Schnitt entlang der Linie IV-IV der Fig. 1 im Bereich der Klemmbacken 31 der Wippen 29. Weil im zusammengesetzten Zustand der beiden Kupplungsteile 1 und 2 der hohle Vorsprung 38 die den Klemmbacken 31 abgewandten Enden der Wippen 29 entgegen der Rückführkraft der Federelemente 33 radial nach innen presst, liegen die Klemmbacken 31 nicht oder nur sehr leicht auf der Aussenseite des Schlauchstükkes 11 an. Der Durchfluss der Flüssigkeit durch das Schlauchstück 11 ist somit in keiner Weise beeinträchtigt.

Wird der zweite Kupplungsteil 2 vom ersten Kupplungsteil 1 abgetrennt, so können die Federelemente 33 die den Klemmbacken 31 abgewandten Enden der Wippen 29 radial nach aussen drükken. Dies bewirkt, dass die beiden Klemmbacken 31 sich radial nach innen bewegen, bis das Schlauchstück 11 vollständig zusammengepresst ist, wie dies in der Fig. 5 gezeigt ist, die einen Schnitt an der gleichen Stelle wie die Fig. 4 darstellt. Damit der Bereich des Schlauchstückes 11 zwischen den Klemmbacken 31 nicht seitlich ausweichen kann, sind an der Stirnseite des dritten Abschnittes 24 des Ansatzes 4 diametral gegenüberliegende Vorsprünge 53 angeordnet. Diese Vorsprünge 53 sind dafür verantwortlich, dass das zusammengepresste Schlauchstück 11 zwischen den Klemmbacken 31 verbleibt.

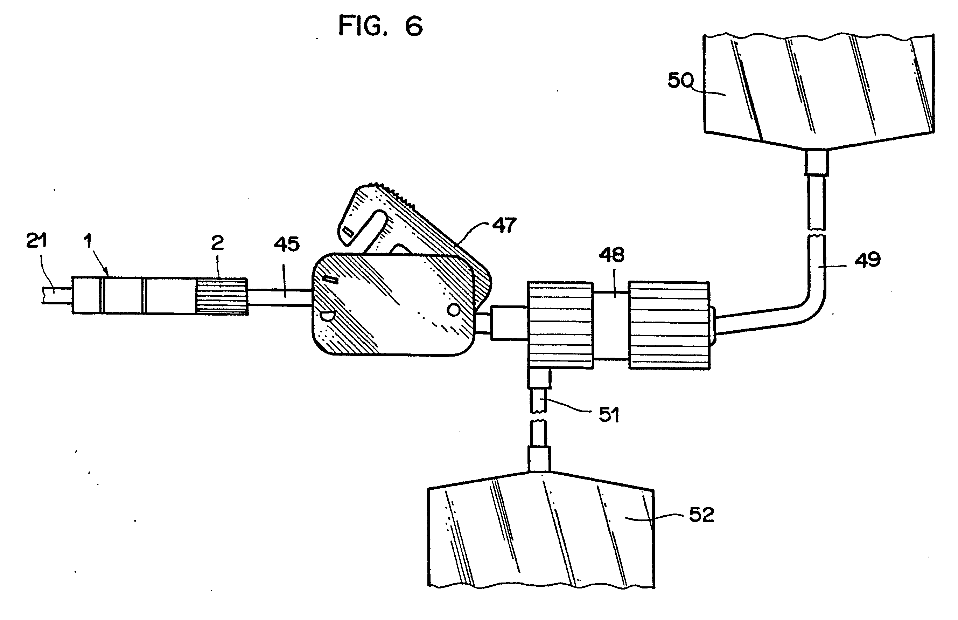

In der Fig. 6 ist ein System zum Ausführen einer Peritonealdialyse bei Patienten mit geschädigten Nieren dargestellt, in welchem die oben beschriebene zweiteilige Kupplungseinrichtung eingesetzt ist. In dieser Figur ist mit 21 ein Teil eines in der Bauchhöhle des Patienten eingelegten Katheters bezeichnet, welcher Teil mit dem Kupplungsteil 1 verbunden ist. Mit 45 ist ein Anschlussschlauch, mit 47 ein Gerät zum Klemmen und Trennen des Anschlussschlauches 45, mit 48 ein Dreiwegverteiler, mit 49 ein Zuführungsschlauch zum Zuführen des frischen Dialysats aus einem Beutel 50 und mit 51 ein Abführungsschlauch zum Abführen des verbrauchten Dialysats in den Beutel 52 bezeichnet. Die von den geschädigten Nieren des Patienten ausgeschiedenen Stoffwechselprodukte werden aus der Bauchhöhle des Patienten durch das verbrauchte Dialysat abgeführt, wonach in die Bauchhöhle des Patienten in umgekehrter Richtung frisches Dialysat eingeführt wird.

Der Durchflusskanal durch die Kupplungseinrichtung und der Anschlussschlauch haben über ihre gesamten Längen einen gleichbleibenden lichten Durchmesser. Geht man von einem konstanten Durchmesser von 3 mm im Tenckhoff-Katheter aus, so hat der Durchflusskanal in der Kupplungseinrichtung den gleichen Durchmesser von 3 mm. Dadurch bleibt die Auslaufgeschwindigkeit des verbrauchten Dialysats überall konstant, was eine wichtige Voraussetzung dafür ist, dass alle Festkörper, die im Tenckhoff-Katheter transportiert werden in gleicher Weise durch die Kupplungseinrichtung hindurch weitergeführt werden. Des weiteren wird dadurch Luft, die während des Kupplungsvorganges in den Durchflusskanal gelangt, mit dem ausfliessenden, verbrauchten Dialysat zuverlässig evakuiert.

Bei einem weiteren Ausführungsbeispiel der erfindungsgemässen Kupplungseinrichtung kann das Schlauchstück 11 der distale Endbereich des oben genannten Katheters 21 sein. In diesem Fall kann auf den Stützring 18 des Ausführungsbeispieles nach der Fig. 1 verzichtet werden. Bei einem dritten Ausführungsbeispiel können die beiden Kupplungsteile zum Reduzieren der Dicke der Kupplungseinrichtung einen im wesentlichen rechteckigen Querschnitt aufweisen. Anstelle des Aussen- und Innengewindes weisen dann die beiden Kupplungsteile zusammenarbeitende Schnappverschlüsse auf, die gegebenenfalls verriegelbar sind. Dadurch wird ein unbeabsichtigtes Trennen der Kupplungsteile vermieden.

## Ansprüche

1. Zweiteilige Kupplungseinrichtung zum Austausch von flüssigen oder gasförmigen Medien, mit einem ersten (1) und einem zweiten (2) Kupplungsteil, wobei die beiden Kupplungsteile voneinander lösbar sind, ein die gesamte Kupplungseinrichtung zentral durchsetzender Durchflusskanal vorhanden ist, im Bereich ihrer äusseren Enden je eine Schlauchleitung angeschlossen ist, der erste Kupplungsteil (1) eine Muffe (3) mit einem zentralen Längsdurchlass (13) und einem wenigstens einmal abgesetzten, achsial verlaufenden Ansatz (4) sowie eine den Ansatz wenigstens teilweise umgehende Hülse (5) und ein erster Teil (6) eines lösbaren Befestigungsmittel der beiden Kupplungsteile (1,2) umfasst, ein elastischer Hohlkörper (11) vorhanden ist, dessen eines Ende in der Hülse (5) befestigt ist, und der zweite Kupplungsteil (2) ein mit dem ersten Teil (6) des Befestigungsmittels zusammenwirkender zweiter Teil (37) des lösbaren Befestigungsmittels und einen sich in Richtung zum ersten Kupplungsteil (1) erstreckenden hohlen Vorsprung (38) aufweist, der den ganzen Ansatz (4) der Muffe und einen Teil der Hülse (5) umgibt, dadurch gekennzeichnet, dass der Hohlkörper (11) wenigstens einen Abschnitt des Durchflusskanals bildet, und dass bewegbare Mittel (29, 30, 33) zum Zusammenpresen des Hohlkörpers (11) ausserhalb des Hohlkörpers derart vorhanden sind, dass der Durchtritt der Medien durch den Hohlkörper gesperrt ist, wenn der erste Kupplungsteil (1) vom zweiten getrennt ist.

2. Kupplungseinrichtung nach Anspruch 1, dadurch gekennzeichnet, dass der erste Teil des Befestigungsmittels ein an einer achsialen Verlängerung (7) der Hülse (5) angeordnetes Aussengewinde (6) und dass der zweite Teil des Befestigungsmittels ein im zweiten Kupplungsteil (2) angeordnetes Innengewinde (37) ist.

3. Kupplungseinrichtung nach Anspruch 2, dadurch gekennzeichnet, dass das andere Ende des Hohlkörpers (11) in der Muffe (3) befestigt ist, dass der Ansatz (4) der Muffe (3) drei Abschnitte aufweist, dass der direkt an die Muffe (3) anschliessende erste Abschnitt (22) einen kleineren Durchmesser als die Muffe (3) und eine Umfangsnut (25) mit einem darin angeordneten Dichtungsring (26) aufweist, dass der an den ersten Abschnitt (22) angrenzende zweite Abschnitt (23) einen kleineren Durchmesser als der erste Abschnitt (22) besitzt, dass der an den zweiten Abschnitt (23) anschliessende dritte Abschnitt (24) einen kleineren Durchmesser als der erste Abschnitt (22) und einen grösseren Durchmesser als der zweite Abschnitt (23) aufweist, und dass im dritten Abschnitt (24) zwei diametral gegenüberliegende, achsial und radial verlaufende Ausnehmungen (27) vorhanden sind, in denen ein Teil der automatischen Sperrmittel (29, 30, 33) angeordnet ist.

4. Kupplungseinrichtung nach Anspruch 2 oder 3, dadurch gekennzeichnet, dass die automatischen Sperrmittel (29, 30, 33) zwei einander gegenüberliegende angeordnete Wippen (29) und wenigstens ein auf das eine Ende jeder Wippe (29) einwirkendes Federelement (33) umfasst und dass das andere Ende jeder Wippe als Klemmbacke (31) zum Zusammenpressen des genannten Hohlkörpers ausgebildet ist.

5. Kupplungseinrichtung nach den Ansprüchen 3 und 4, dadurch gekennzeichnet, dass benachbart zu den Ausnehmungen (27) im dritten Abschnitt (24) quer zu den Ausnehmungen verlaufende Einschnitte (28) vorhanden sind, dass die Wippen (29) je zwei Lagerzapfen (30) aufweisen und dass die Lagerzapfen (30) in den genannten Einschnitten (28) gelagert sind.

6. Kupplungseinrichtung nach Anspruch 5, dadurch gekennzeichnet, dass an der Stirnseite des dritten Abschnittes (24) des Ansatzes (4) zwei einander gegenüberliegende Vorsprünge (53) befestigt sind und dass die Vorsprünge (53) zum Vermeiden des Ausweichens des Hohlkörpers (11) beim Zusammenpressen durch die Klemmbacken (31) seitlich derselben angeordnet sind.

7. Kupplungseinrichtung nach einem der Ansprüche 1 bis 6, dadurch gekennzeichnet, dass der erste Kupplungsteil vorwiegend aus einem inerten Metall, z.B. Titan, besteht und dass der elastische Hohlkörper (11) ein Schlauchstück aus Silikon ist.

8. Kupplungseinrichtung nach einem der Ansprüche 1 bis 6, dadurch gekennzeichnet, dass der elastische Hohlkörper das distale Ende eines Katheters (21) ist.

9. Einrichtung zum Ausführen einer Peritonealdialyse bei Patienten mit geschädigten Nieren mit einer Kupplungseinrichtung nach einem der vorangehenden Ansprüche.

**Claims**

1. A two-piece coupling device for the exchange of liquid or gaseous media, having a first (1) and a second (2) coupling part, the two coupling parts being detachable from one another, a flow-through channel running through the centre of the whole of the coupling device, one hose line each being connected in the region of their outer ends, the first coupling part (1) including a sleeve (3) with a longitudinal central passage (13) and an axially projecting extension (4) which is stepped at least once, a jacket (5) which at least partially surrounds said extension and a first part (6) of a detachable fastening means of the two coupling parts (1, 2), a resilient hollow body (11) secured at one end in the jacket (5) and the second coupling part (2) having a second part (37) of the detachable fastening means cooperating with the first part (6) of the fastening means and a hollow projection (38) extending towards said first coupling part (1), said projection entirely surrounding said extension (4) of the sleeve and a part of the jacket (5), characterized in that the hollow body (11) forms at least one section of the flow-through channel and in that moveable means (29, 30, 33) for compressing the hollow body (11) are located outside the hollow body in such a way that the media are prevented from flowing through the hollow body when the first coupling part (1) is separated from the second.

2. The coupling device according to claim 1, characterized in that the first part of the fastening means is an external thread (6) disposed on an axial prolongation (7) of the jacket (5) and in that the second part of the fastening means is an internal thread (37) within the second coupling part (2).

3. The coupling device of claim 2, characterized in that the other end of the hollow body (11) is secured in the sleeve (3), in that the extension (4) of the sleeve (3) has three sections, in that the first section (22) directly adjacent to the sleeve (3) has a smaller diameter than the sleeve (3) and a circumferential groove (25) with a sealing ring (26) disposed therein, in that the second section (23) adjacent to the first section (22) has a smaller diameter than the first section (22), in that the third section (24) adjacent to the second section (23) has a smaller diameter than the first section (22) and a larger diameter than the second section (23), and in that there are two diametrically opposed axially and radially extending recesses (27) in the third section (24), a part of the automatic blocking means (29, 30, 33) being disposed in said recesses.

4. The coupling device of claim 2 or 3, characterized in that the automatic blocking means (29, 30, 33) comprise two oppositely disposed rockers (29) and at least one spring element (33) acting on one end of each rocker (29) and in that the other end of each rocker is designed as a clamping jaw (31) to compress the said hollow body.

5. The coupling device of claims 3 and 4, characterized in that adjacent to the recesses (27) in the third section (24) there are notches (28) running at right angles to the recesses, in that the rockers (29) comprise two pivot pins (30) respectively and in that the pivot pins (30) are disposed in the said notches (28).

6. The coupling device of claim 5, characterized in that there are two opposed projections (53) fixed to the end face of the third section (24) of the extension (4) and in that the projections (53) are disposed to prevent a lateral displacement of the hollow body (11) upon compression thereof by the clamping jaws (31).

7. The coupling device of one of claims 1 to 6, characterized in that the first coupling part is made predominantly of an inert metal, e.g. titanium, and in that the resilient hollow body (11) is a tubing connection of silicone.

8. The coupling device of one of claims 1 to 6, characterized in that the resilient hollow body is the distal end of a catheter (21).

9. A device for carrying out peritoneal dialysis in patients with damaged kidneys, with a coupling device according to one of the preceding claims.

**Revendications**

1. Accouplement à deux pièces pour l'échange de fluides comprenant une première partie d'accouplement (1) et une deuxième partie d'accouplement (2), ces deux parties étant séparables l'une de l'autre, un canal de circulation central étant établi pour traverser toute la longueur de l'accouplement, un conduit à tuyau étant raccordé à chacune des deux extrémités extérieures de l'accouplement, la première partie d'accouplement (1) comprenant un manchon (3) avec un passage central longitudinal (13) et une réduction (4) s'étendant axialement avec au moins une fois un rétrécissement, de même qu'une douille (5) entourant au moins partiellement la réduction et une première partie (6) d'un moyen de fixation amovible des deux parties d'accouplement (1,2), un corps creux élastique (11) ayant une de ses extrémités fixée à la douille (5) et la deuxième partie d'accouplement (2) présentant une deuxième partie (37) du moyen de fixation amovible coopérant avec la première partie (6) du moyen de fixation, et un ravancement creux (38) s'étendant dans la direction de la première partie d'accouplement (1) et entourant toute la réduction (4) du manchon (3) de même qu'une portion de la douille (5), caractérisé en ce que le corps creux (11) forme au moins un tronçon du canal de circulation, et en ce que des moyens mobiles (29, 30, 33) pour comprimer le corps creux (11) sont disposés à l'extérieur de ce corps creux, de manière telle que la section du flux à travers le corps creux soit obturée lorsque la première partie d'accouplement (1) est séparée de la deuxième.

2. Accouplement selon la revendication 1, caractérisé en ce que la première partie du moyen de fixation est un filetage extérieur (fixe) disposé sur une prolongation axiale (7) de la douille (5) et en ce que la deuxième partie du moyen de fixation est un filetage intérieur (37) disposé dans la deuxième partie d'accouplement (2).

3. Accouplement selon la revendication 2, caractérisé en ce que l'autre extrémité du corps creux (11) est fixée dans le manchon (3), en ce que la réduction (4) du manchon (3) présente trois tronçons, le premier tronçon (22) directement connecté au manchon (3) présentant un plus petit diamètre que le manchon (3) et ayant une gorge périphérique (23) dans laquelle est disposé un anneau d'étanchéité (26), le deuxième tronçon (23) jointif au premier tronçon (22) ayant un diamètre plus petit que celui du premier tronçon (22), et le troisième tronçon (24) jointif au deuxième tronçon (22) ayant un diamètre plus petit que celui du premier tronçon (22) mais plus grand que celui du deuxième tronçon (23), deux évidements (27) diamétralement opposés et s'étendant axialement et radialement étant ménagés dans le troisième tronçon (24), des moyens de verrouillage automatique (29, 30, 33) étant disposés dans ces évidements (27).

4. Accouplement selon la revendication 2 ou la revendication 3, caractérisé en ce que les moyens de verrouillage automatique (29, 30, 33) comprennent deux bascules (29) disposées en face l'une de l'autre et au moins un élément ressort (33) agissant sur une extrémité de chaque bascule (29), l'autre extrémité de chaque bascule étant formée comme joue de pinçage (31) pour comprimer ledit corps creux.

5. Accouplement selon les revendications 3 et 4, caractérisé en ce que des entailles (28) sont ménagées dans le troisième tronçon (24) au voisinage des évidements (27), perpendiculairement à ceux-ci, les bascules (29) présentant chacune deux tétons d'appui pivotant (30) qui viennent en engagement de palier dans lesdites entailles (28).

6. Accouplement selon la revendication 5, caractérisé en ce que deux projections (53) se faisant face sont fixement liées à la face frontale du troisième tronçon (24) de la réduction (4), ces projections (53) servant à éviter un fluage latéral du corps creux (11) lors de sa compression par les joues de serrage (31).

7. Accouplement selon l'une des revendications 1 à 6, caractérisé en ce que la première partie d'accouplement est faite principalement d'un métal inerte, par exemple le titane, le corps creux élastique (11) étant une pièce de tuyau de silicone.

8. Accouplement selon l'une des revendications 1 à 6, caractérisé en ce que le corps creux élastique est l'extrémité distale d'un cathéter (21).

9. Installation pour l'exécution d'une dialyse péritonéale auprès de patients ayant les reins abîmés, comprenant un dispositif d'accouplement selon une des revendications précédentes.

FIG. 1

FIG. 2

EP 0 263 789 B1

FIG. 3

FIG. 4

FIG. 5

FIG. 6